# EUROPEAN PATENT APPLICATION

(11) **EP 1 661 575 A1**
(43) Date of publication of application: **31.05.2006**
(21) Application number: 04106118.5
(22) Date of filing: 26.11.2004
(51) Int. Cl.: A61K 33/08, A61K 9/48, A61K 9/24, A61K 9/26, A61P 15/08

(54) **Use of magnesium in the treatment of disorders related to hormonal variations in women**

(71) Applicant: ZAMBON GROUP S.p.A., 36100 Vicenza (IT)
(72) Inventor: Porchetti, Anna, 20100, Milano (IT); Porzio, Stefano, 22077, Olgiate Comasco (IT); Costa, Andrea, 20090, Trezzano sul Naviglio (IT); Penci, Marisa, 20090, Trezzano sul Naviglio (IT)
(74) Representative: Longoni, Alessandra

(57) **Abstract**

The present invention relates to a novel treatment of the symptoms associated with disorders related to normal hormonal variations in women and particularly it provides a pulsatile release formulation of magnesium for treating said symptoms.

## Description

The present invention relates to a novel treatment of the symptoms associated with disorders related to normal hormonal variations in women and particularly it provides a pulsatile release formulation of magnesium for treating said symptoms.

The most common disorders relating to normal variation of the sex hormone cycle of women include depressed mood, anxiety/tension, mood swing, irritability, decrease interest, concentration difficulties, fatigue, bloating, headache, migraine, appetite changes, insomnia/hypersomnia, feeling out of control and physical symptoms.

Particularly, premenstrual syndrome (hereinafter PMS) is a disorder that includes a broad group of emotional, behavioural and physical symptoms that occur cyclically before and during the menstrual period.

Lists of symptoms commonly reported by women suffering of the above pathologies are known and available through bibliographic sources.

The pharmacological treatment that has been tried comprises serotonin re-uptake inhibitors, diuretics, hormonal treatment as well as dietary supplementation of vitamins and mineral and natural products.

Generally, non-steroidal anti-inflammatory drugs are administered to PMS patients but they are only effective for some of the physical symptoms.

Most of the compounds used for the treatment are limited due to side effects.

Magnesium is an important constituent of all soft tissues and bones.

Next to potassium it is the predominant cation in the cell and is an essential part of many enzyme systems. It is important in maintaining function in nerves and muscles.

The importance of this element in various disorders has been recognized.

Low levels of magnesium occur in most of the disorders associated with normal hormone variation in women during fertile, peri and post menopausal age.

There is no evidence that larger than normal intakes are harmful.

Magnesium deficiency has been indicated as a possible factor in some of the symptomatology of premenstrual tension (Nicholas, International Symposium de Clinique Humaine, Paris 1973, Springer Verlag 261-263).

The apparent magnesium deficiency in PMS may depend upon decreased intake or increased excretion and depletion.

Posaci et al. (Acta Obset. Gynecol. Scand. 1994, 73, 452-455) performed an analysis of magnesium levels in patients suffering of premenstrual tension. It resulted that the mean magnesium levels was significantly lower in PMS patients than in control group.

These data confirm that magnesium deficiency plays an important role in the etiology of PMS and, consequently, magnesium supplement may be useful in the treatment of this syndrome.

US 5,612,061 describes a composition for the treatment of premenstrual syndrome comprising an effective amount of calcium, magnesium, an analgesic and a diuretic sufficient to reduce the symptoms of PMS.

Magnesium supplementation has been documented to relieve certain symptoms of PMS including pain and mood fluctuations (Oral Magnesium successfully relieves premenstrual mood changes F. Facchinetti et al. Obstetrics & Gynecology 1991, vol. 78, No. 2, pages 177-181).

Daily supplement of 200 mg of magnesium reduced mild premenstrual symptoms of fluid retention (Walker et al. Journal of Women's Health, 1998, vol. 7, No. 9, 1157-1165).

Other trials proved the efficacy of magnesium supplementation alone or in combination with vitamin B6 in PMS.

FR 2709962 describes compositions comprising magnesium and vitamin B6 for the prevention or the treatment of premenstrual syndrome.

In a wide review of literature recently published (Rapkin A., Psyco Neuro Endocrinology 2003, vol. 28, pages 39-53) both pharmacological and not pharmacological treatments of PMS were evaluated. Not pharmacologic management with some evidence for efficacy includes magnesium, vitamin B6, L-triptophan supplementation or a complex carbohydrate drink.

It has been observed that most of the hormones whose normal variations may cause disorders in women during fertile, peri and post menopausal age, foresee a circadian release.

For example, many studies have demonstrated that gonadotropin release occurs not only with an ultradiurn profile but also shows a daily profile.

Particularly, in healthy women with normal menstrual cycle, a 24 hours rhythm of estradiol was proved. Several studies were performed showing interindividual differences from which an average range of onset between 4 a.m. and 8 a.m. appears to be statistically confirmed.

Moreover, in healthy women with normal menstrual cycle, a 24 hours rhythm of luteinizing hormone (LH) was proved. The peak of release of the hormone occurs in the night-early morning during the luteal phase, ranging from 8 p.m. to 2 a.m. (Mitamura et al., The Journal of Clinical Endocrinology and Metabolism, 2000, vol 85, No. 3).

Melatonine has a circadian rhythm of release. During the night, some neurons release noradrenalin in the interstitium. Noradrenalin stimulates melatonin synthesis through cAMP accumulation. Melatonin release is higher during the dark time of the day, with peak observed at 2 a.m. (Parry et al. Arch. Gen. Phychiatry 1990, vol 47, 1139-1146).

Circadian patterns of prolactin change during the four phases of the menstrual cycle being higher in the luteal phase. In a consistent percentage of individuals serum prolactin rises notably during the night. Some studies indicate a first peak between 4-5 a.m. and an evening peak at about 8-10 p.m. (Tennekoon KH, Lenton EA, Journal Reprod. Fertil. 1985, 73(2), 523-7; Brzezinski A, J. Clin. Endocrinol. Metab. 1988, 66(5), 891-5; Webley and Lenton Fertil. Steril. 1987, 48(2), 218-222; Carandente et al., Chronobiologia 1989, 16(1), 35-44).

It has now surprisingly found that the administration of magnesium in a chronomodulated manner, which mimics the circadian rhythms of the normal hormonal cycle in women, is able to manage the symptoms associated with disorders related to said normal variations.

Therefore, it is an object of the present invention a new method for preventing or treating symptoms associated with disorders related to normal hormonal variations in women, which comprises a pulsed release administration of a therapeutically effective amount of magnesium.

In other words, it is an object of the present invention the use of magnesium in the preparation of a pulsed release pharmaceutical or nutritional composition for the prevention or treatment of the symptoms associated with the disorders related to normal hormonal variations in women, preferably disorders associated with premenstrual syndrome.

According to the present invention the term "magnesium" refers to sources of magnesium ions for oral ingestion, which comprise chelated combinations such as, for example, magnesium α-ketogluconato, aspartate, glycinate, lysinate, orotate and taurate, and magnesium salts such as, for example, magnesium sulphate, phosphate, oxide, hydroxide, citrate, carbonate, lactate, glycerophosphate, bicarbonate and chloride.

Preferably, the magnesium source is selected from pharmaceutically acceptable salts of magnesium such as, for example magnesium hydroxide, carbonate, sulphate, chloride, gluconate, phosphate, oxide and other salts suitable for human administration or a combination thereof.

Still more preferred are high magnesium content salts such as magnesium hydroxide and oxide, magnesium oxide being particularly preferred.

The dietary or therapeutic doses of magnesium may range from 100 to 360 mg a day, preferably from 200 to 250 mg.

According to the present invention the term"to treat" or "treating" refers to the prevention and/or any reduction in the symptoms associated with the disorders related to normal hormonal variations in women.

According to the present invention the term "pulsed or pulsatile release" means, unless otherwise specified, a pulsatile release of magnesium, wherein the magnesium is released in spaced apart"pulses".

To the best of inventors' knowledge, the concept of using spaced drug delivery systems for magnesium, wherein magnesium is released immediately initially and then as a pulse at a predetermined time interval to meet the need of mimic the circadian variations of the normal hormonal cycle in women is neither known in the art nor suggested by any prior art reference. By incorporating both an immediate release dosage unit and a delayed release dosage unit of magnesium, the composition according to the invention mimics a biphasic dosing profile without repeated dosing , i.e., with only a single administration.

In particular, the composition according to the invention provides a first immediate release and a second delayed release of magnesium after a delay of about 2-3 hours following administration.

Generally, upon ingestion of the dosage form magnesium immediate release occurs within about 15 to 60 minutes.

It follows a magnesium release-free time interval wherein a little amount of magnesium or no magnesium may be released depending upon the dosage form design including with this term delaying release means, sources of magnesium ion suitable for oral ingestion and so on.

The second delayed release of magnesium then occurs.

In a preferred aspect, when the composition is administered late in the afternoon, e.g. between 8 and 9 p.m., it provides a twice daily dosing profile of magnesium so that a first amount of magnesium is available concomitantly nocturnal/early diurnal highest release melatonin and a second amount of is available later, concomitantly the early diurnal highest release of estrogens and prolactin.

This biphasic pulsed release profile awards favourable properties to the composition because it allows a delivery of magnesium which may synergise with the beneficial effects of the maximum daily release of melatonin and also may prevent and treat the problems caused by magnesium depletion, such as, e.g., migraine attacks and cerebral vasospasms, occurring concomitantly the maximal activity of estrogens and prolactin in the women.

It is therefore another object of the present invention an oral pharmaceutical composition which comprises magnesium as an active agent in admixture with pharmaceutically acceptable carriers allowing the delivery of the magnesium in a pulsed manner as indicated above.

An oral nutritional composition which comprises magnesium in admixture with nutritional acceptable carriers allowing the delivery of the magnesium in a pulsed manner is also encompassed by the present invention.

In particular, the compositions of the present invention comprise:
(i) an immediate release dosage unit comprising a first dose of magnesium that is released substantially immediately following the oral administration of the composition;
(ii) a delayed release dosage unit comprising a second dose of magnesium and a means for delaying release of the second dose resulting in the second delivery at approximately 2-3 hours following the oral administration of the composition.

Preferably, from 40 to 80% by weight, more preferably from 50 to 70% by weight of the total amount of magnesium in the composition according to the invention is released in the first pulse; and correspondingly from 20 to 60% by weight, more preferably from 30 to 50% by weight is released in the second pulse.

According to the present invention, the immediate and delayed release portions taken together, provide the total administrable dose of magnesium ranging from 100 mg to 400 mg, preferably from 200 mg to 300 mg.

The individual immediate and delayed dosage units of the composition according to the invention, may be formulated, for example, as tablets, beads, granules or particle.

The above-mentioned tablets, beads, granules or particles of different magnesium release profiles (e.g., immediate and delayed release profiles) may be mixed and included in a capsule, tablet or matrix to provide a dosage form having the desired pulsatile release profile.

According to a preferred aspect of the present invention, the individual dosage units may be formulated in two separate layers which can be compacted in a dosage form of a tablet. For example, magnesium-containing beads, granules or particles with immediate and delayed release profile can be compressed into two different layers and then compacted together into a single tablet using conventional tabletting procedures.

According to another aspect of the present invention, a dosage form, e.g. a tablet, is provided, which comprises an inner magnesium-containing core and at least one magnesium-containing layer surrounding the inner core. The dosage form has an outer layer, corresponding to the immediate release dosage unit that releases drug substantially immediately following oral administration and an inner core, corresponding to the delayed release dosage unit.

According to a further aspect of the invention, capsules housing two magnesium-containing dosage units, wherein each dosage unit may comprise one or more tablet, wherein each tablet within the capsule provides an immediate or delayed magnesium release profile, are also provided. For example, one or more tablets release magnesium substantially immediately following ingestion of the dosage form, while one or more tablets release magnesium according to the desired delayed release profile.

The delayed release dosage unit comprises a means suitable for delaying said second dose. Coating materials may be useful for effecting such delayed release. Examples of suitable coating materials include, but are not limited to, cellulose polymers such as cellulose acetate phthalate, hydroxypropyl cellulose, hydroxypropyl methylcellulose, hydroxypropyl methylcellulose phthalate and hydroxypropyl methylcellulose acetate succinate; Shellac gum; polyvinyl acetate phthalate, acrylic acid polymers and copolymers, and methacrylic resins.

Preferred coating materials are cellulose acetate phthalate, Shellac gum and hydroxypropyl methylcellulose

Hydroxypropyl methylcellulose being the preferred one.

Optional components present in the individual magnesium-containing dosage units include, but are not limited to, diluents, binders, lubricants, disintegrants, stabilizers, dissolution accelerators, surfactants and colouring agents. Diluents are typically necessary to increase the bulk of a tablet so that a practical size is provided for compression. Suitable diluents include, for example, dicalcium phosphate, calcium sulfate, lactose, cellulose, kaolin, mannitol, sodium chloride, dry starch, hydrolyzed starches, silicon dioxide, titanium oxide, alumina, talc, microcrystalline cellulose, and powdered sugar. Binders are used to impart cohesive qualities to a tablet formulation, and thus ensure that a tablet remains intact after compression. Suitable binder materials include, but are not limited to, starch (including corn starch and pregelatinized starch), gelatin, sugars (including sucrose, glucose, dextrose, lactose and sorbitol), polyethylene glycol, waxes, natural and synthetic gums, e.g., acacia, tragacanth, sodium alginate, polyvinylpyrrolidone, celluloses, and synthetic polymers such as polymethacrylates and polyvinylpyrrolidone. Lubricants are used to facilitate tablet manufacture; examples of suitable lubricants include, for example, magnesium stearate, calcium stearate, stearic acid, glyceryl behenate, and polyethylene glycol. Disintegrants are used to facilitate tablet disintegration or "breakup" after administration, and are generally starches, clays, celluloses, algins, gums or cross linked polymers. Stabilizers are used to inhibit or retard drug decomposition reactions which include, by way of example, oxidative reactions. Dissolution accelerators include, for example, sodium croscarmellose, sodium carboxymethylcellulose, starch, sodium starch glycolate, dicalcium phosfate.

Surfactants may be anionic, cationic, amphoteric or nonionic surface active agents, with anionic surfactants preferred. Suitable anionic surfactants include, but are not limited to, those containing carboxylate, sulfonate and sulfate ions, associated with cations such as sodium, potassium and ammonium ions. Colouring agents include, for example, carminic acid (E 120), Ponceau 3R (E124), Tartrazine (E102), Sunset yellow FCF (E110).

The preparation of the compositions according to the present invention may be carried out following methods known to the skilled in the art by using well known and readily available ingredients and by means of standard machineries.

For example, the immediate release dosage unit may be formulated as one or more tablets, a plurality of magnesium-containing beads, granules or particles, or an outer layer of a coated core dosage form contains a therapeutically effective quantity of the active agent with conventional pharmaceutical excipients. A preferred method for preparing immediate release tablets is by compressing a magnesium-containing blend, e.g., blend of granules, prepared using a direct blend, wet-granulation or dry-granulation process. A preferred method for forming immediate release drug-containing blend is to mix drug particles directly with one or more excipients such as diluents, binders, disintegrants, lubricants, glidants, colorants or the like.

For example, the delayed release dosage units can be prepared by coating magnesium or a magnesium-containing compositions, e.g., tablets, beads, granules or particles, with a selected coating material as defined above. When a coating is used to provide delayed release dosage units, particularly preferred coating materials comprise cellulose polymers, especially hydroxypropyl methylcellulose. The delayed release dosage unit may be coated using conventional techniques well known to a person skilled in the art.

Alternatively, a delayed release unit may be formulated by dispersing the magnesium within a matrix of a suitable material such as a hydrophilic polymer or a fatty compound. The hydrophilic polymers may be comprised of polymers or copolymers of cellulose, cellulose ester, acrylic acid, methacrylic acid, methyl acrylate, ethyl acrylate, and vinyl or enzymatically degradable polymers or copolymers as described above. These hydrophilic polymers are particularly useful for providing a delayed release matrix. Fatty compounds for use as a matrix material include, but are not limited to, waxes (e.g. carnauba wax) and glycerol tristearate. Once the magnesium is mixed with the matrix material, the mixture can be compressed into tablets.

In an alternative embodiment of the present invention, the immediate release dosage unit and the delayed release dosage unit may comprise magnesium salts with different aqueous solubility, a soluble portion and an insoluble portion respectively.

The soluble portion comprising a single or a mixture of soluble magnesium salt(s), covering a range of ionized magnesium according to the invention.

The insoluble portion comprising a single or a mixture of less soluble magnesium salt(s), covering a range of ionized magnesium concentration according to the invention.

Suitable magnesium water and acid soluble salts are magnesium citrate, gluconate, glycerophosphate, phosphate, lactate, hydroxide, oxide, sulphate or other salts suitable for human administration.

They will ensure an average and quick dissolution and an absorption profile within 5-6 hours as reported in the literature.

The insoluble portion may be constituted by magnesium oxide or any other salt with low water and acid solubility.

It is, preferably, constituted by soluble salt or a mixture of salts coated by a layer which is able to delay the dissolution of magnesium.

Thus, it is evident how the source of magnesium may be different for the two above mentioned portions of the composition or, preferably, the source of magnesium is the same keeping the dissolution profile in the ranges of the invention.

Practical dosage forms of the compositions according to the invention are, for example, tablets constituted by a delayed release core, comprising the magnesium, coated by a release delaying agent and further coated by an outer immediate release layer comprising the same active agent optionally coming from an alternative sources of magnesium; tablets obtained by the compression of two layer tablets constituted by an immediate release layer and a delayed release layer; or capsulses housing immediate release beads, particles, granules or tablets and delayed release beads, particles, granules or tablets.

The preferred dosage form of the composition of the invention is a tablet.

It is to be understood that while the invention is described in conjunction of the preferred embodiments thereof, those skilled in the art are aware that other embodiments could be made without departing from the spirit of the invention.

For better illustrating the invention the following examples are provided.

The following formulation examples are only illustrative and are not intended to limit the scope of the present invention.

### Example 1

### Immediate release unit of a pulsatile release composition

The colouring agent in a portion of microcrystalline cellulose is previously dispersed.

All the ingredients were mixed, magnesium stearate excluded, in a biconic mixer for ten minutes.

At the end of the operation, magnesium stearate was added and the mixture was mixed for further three minutes.

The so obtained mixture, depending on the amounts of the used excipients may have a composition comprised in the following ranges:

| | |
|---|---|
| Magnesium oxide | 35-45% by weight. |
| Dicalcium phosphate | 18-27% by weight. |
| Microcrystalline cellulose | 23-28% by weight. |
| Sodium croscarmellose | 1-3% by weight. |
| Silicium bioxide | 1-8% by weight. |
| Magnesium stearate | 0,5-2% by weight. |
| Colouring agent being the total amount 100%. | 0-0,1% by weight. |

### Example 2

### Delayed release unit of a pulsatile release composition

All the ingredients were mixed, magnesium stearate excluded, in a biconic mixer for ten minutes.

At the end of the operation, magnesium stearate was added and the mixture was mixed for further three minutes.

The so obtained mixture, depending on the amounts of the used excipients may have a composition comprised in the following ranges:

| | |
|---|---|
| Magnesium oxide | 40-55% by weight. |
| Dicalcium phosphate | 12-25% by weight. |
| Microcrystalline cellulose | 10-25% by weight. |
| Hydroxypropylmethylcellulose | 10-25% by weight. |
| Silicium bioxide | 1-2,5% by weight. |
| Magnesium stearate | 0,5-2% by weight. |
| Colouring agent being the total amount 100%. | 0-0,1% by weight. |

### Example 3

### Preparation of a pulsatile release magnesium composition as a tablet

Tablets were prepared by conventional compression methods.

The mixtures obtained in examples 1 and 2 were charged in two different measuring chutes of the tablet making machine (Manesty LP) controlling in process weight and hardness of tablets.

### Example 4

### Preparation of a pulsatile release magnesium composition as a capsule

Capsules were prepared by conventional methods.

The mixtures obtained in examples 1 and 2 were filled into capsules optionally in admixture with suitable excipients.

### Example 5

By working according to what described in example 3 the following tablets were obtained:

**Tablet 1:**

| | |
|---|---|
| Magnesium oxide | 45% by weight. |
| Dicalcium phosphate | 22% by weight. |
| Microcrystalline cellulose | 21% by weight. |
| Hydroxypropylmethylcellulose | 5% by weight. |
| Silicium bioxide | 5% by weight. |
| Sodium croscarmellose | 1% by weight. |
| Magnesium stearate | 1% by weight. |
| Colouring agent | <1% by weight. |

**Tablet 2:**

| | |
|---|---|
| Magnesium oxide | 45% by weight. |
| Microcrystalline cellulose | 19% by weight. |
| Dicalcium phosphate | 18% by weight. |
| Hydroxypropylmethylcellulose | 11% by weight. |
| Silicium bioxide | 5% by weight. |
| E 468 | 1% by weight. |
| Magnesium stearate | 1% by weight. |
| Colouring agent | <1% by weight. |

**Tablet 3:**

| | |
|---|---|
| Magnesium oxide | 45% by weight. |
| Microcrystalline cellulose | 20% by weight. |
| Dicalcium phosphate | 17% by weight. |
| Hydroxypropylmethylcellulose | 11% by weight. |
| Silicium bioxide | 5% by weight. |
| Sodium croscarmellose | 1% by weight. |
| Magnesium stearate | 1% by weight. |
| Colouring agent | <1% by weight. |

### Example 6

### Immediate release unit of a pulsatile release composition

The preparation of the granulate was carried out by using usual excipients and conventional preparative techniques.

The colouring agent was dispersed in water (wetting solution).

All the ingredients were mixed in a fast mixer, sodium croscarmellose and magnesium stearate excluded, with the wetting solution for ten minutes.

The obtained granulate was discharge, sieved with two millimetres net, dried under vacuum at 40°C for 12 hours.

At the end of the drying process, it was sieved with 0,7 millimetres net.

It was then mixed with the remaining ingredients for five minutes.

### Example 7

### Delayed release unit of a pulsatile release composition

The preparation of the granulate was carried out by using usual excipients and conventional preparative techniques.

The colouring agent was dispersed in water (wetting solution).

All the ingredients were mixed in a fast mixer, hydroxypropylmethylcellulose and magnesium stearate excluded, with water for ten minutes.

The obtained granulate was discharge, sieved with two millimetres net, dried under vacuum at 40°C for 12 hours.

At the end of the drying process, it was sieved with 0,7 millimetres net.

It was then mixed with the remaining ingredients for five minutes.

### Example 8

### Preparation of a pulsatile release magnesium composition as a tablet.

Tablets were prepared by conventional compression methods by using granulates obtained according to the method described in examples 6 and 7 optionally in admixture with suitable excipients.

### Example 9

### Preparation of a pulsatile release magnesium composition as a capsule.

Capsules were prepared by conventional methods by using granulates obtained according to the method described in examples 6 and 7 optionally in admixture with suitable excipients.

## Claims

1. An oral pulsed release pharmaceutical/nutritional composition which comprises magnesium as active agent in admixture with pharmaceutically/nutritionally acceptable carriers.

2. A composition according to claim 1 wherein the pulsed release mimics the circadian rhythms of normal hormon cycle in women.

3. A composition according to claim 1 and 2 that comprises:
(i) an immediate release dosage unit comprising a first dose of magnesium that is released substantially immediately following the oral administration of the composition;
(ii) a delayed release dosage unit comprising a second dose of magnesium and a means for delaying release of the second dose resulting in the second delivery at approximately 2-3 hours following the oral administration of the composition.

4. A composition according to one of the preceding claims wherein the magnesium source is selected from pharmaceutically acceptable salts of magnesium.

5. A composition according to claim 4 wherein the magnesium source is magnesium oxide.

6. A composition according to claim 3 wherein from 40 to 80% by weight of the total amount of magnesium in the composition is released in the first pulse and correspondingly from 20 to 60% by weight is released in the second pulse.

7. A composition according to claim 6 wherein from 50 to 70% by weight of the total amount of magnesium in the composition is released in the first pulse and correspondingly from 30 to 50% by weight is released in the second pulse.

8. A composition according to claim 3 wherein the immediate and delayed release portions taken together provide a total administrable dose of magnesium ranging from 100 mg to 400 mg.

9. A composition according to claim 8 wherein the portions provide a total administrable dose of magnesium ranging 200 mg to 300 mg.

10. A composition according to claim 3 wherein the means suitable for delaying said second dose are selected from cellulose acetate phthalate, Shellac gum and hydroxypropyl methylcellulose

11. A composition according to claim 10 wherein the mean suitable for delaying said second dose is hydroxypropyl methylcellulose.

12. Use of magnesium in the preparation of a pulsed release pharmaceutical or nutritional composition for the prevention or treatment of the symptoms associated with the disorders related to normal hormonal variations in women.

13. Use of magnesium according to claim 12 wherein said pulsed release pharmaceutical or nutritional composition mimics the circadian rhythms of normal hormon cycle in women.

14. Use of magnesium according to claim 13 for the prevention or treatment of the symptoms associated with premenstrual syndrome.

15. Use of magnesium according to claim 14 wherein the pulsed release pharmaceutical or nutritional composition provides a twice daily dosing profile of magnesium so that a first amount of magnesium is available concomitantly nocturnal/early diurnal highest release melatonin and a second amount of is available later, concomitantly the early diurnal highest release of estrogens and prolactin.

## Amended claims

### Amended claims in accordance with Rule 86(2) EPC.

**1.** An oral pulsed release pharmaceutical or nutritional composition which comprises magnesium as active agent in admixture with pharmaceutically or nutritionally acceptable carriers, comprising:
(i) an immediate release dosage unit comprising a first dose of magnesium that is released substantially immediately following the oral administration of the composition;
(ii) a delayed release dosage unit comprising a second dose of magnesium and a means for delaying release of the second dose resulting in the second delivery at approximately 2-3 hours following the oral administration of the composition.

**2.** A composition according to claim 1 wherein the pulsed release mimics the circadian rhythms of normal hormon cycle in women.

**3.** A composition according to one of the preceding claims wherein the magnesium source is selected from pharmaceutically acceptable salts of magnesium.

**4.** A composition according to claim 3 wherein the magnesium source is magnesium oxide.

**5.** A composition according to claim 1 wherein from 40 to 80% by weight of the total amount of magnesium in the composition is released in the first pulse and correspondingly from 20 to 60% by weight is released in the second pulse.

**6.** A composition according to claim 5 wherein from 50 to 70% by weight of the total amount of magnesium in the composition is released in the first pulse and correspondingly from 30 to 50% by weight is released in the second pulse.

**7.** A composition according to claim 1 wherein the immediate and delayed release portions taken together provide a total administrable dose of magnesium ranging from 100 mg to 400 mg.

**8.** A composition according to claim 7 wherein the portions provide a total administrable dose of magnesium ranging 200 mg to 300 mg.

**9.** A composition according to claim 1 wherein the means suitable for delaying said second dose are selected from cellulose acetate phthalate, Shellac gum and hydroxypropyl methylcellulose

**10.** A composition according to claim 9 wherein the mean suitable for delaying said second dose is hydroxypropyl methylcellulose.

**11.** Use of magnesium in the preparation of a pulsed release pharmaceutical or nutritional composition for the prevention or treatment of the symptoms associated with the disorders related to normal hormonal variations in women.

**12.** Use of magnesium according to claim 11 wherein said pulsed release pharmaceutical or nutritional composition mimics the circadian rhythms of normal hormon cycle in women.

**13.** Use of magnesium according to claim 12 for the prevention or treatment of the symptoms associated with premenstrual syndrome.

**14.** Use of magnesium according to claim 13 wherein the pulsed release pharmaceutical or nutritional composition provides a twice daily dosing profile of magnesium so that a first amount of magnesium is available concomitantly nocturnal/early diurnal highest release melatonin and a second amount of is available later, concomitantly the early diurnal highest release of estrogens and prolactin.
